# EUROPEAN PATENT SPECIFICATION

(11) **EP 0 353 191 B1**
(45) Date of publication and mention of the grant of the patent: **04.12.1996**
(21) Application number: 89810555.6
(22) Date of filing: 20.07.1989
(51) Int. Cl.: C12N 15/56, C12N 9/24, C12P 21/02

(54) **DNA sequences encoding polypeptides having beta-1,3-glucanase activity**
DNS-Sequenzen, die Polypeptiden mit beta-1,3-Glukanase-Aktivität codieren
Séquences d'ADN codant des polypeptides avec activité béta-1,3-glucanase

(30) Priority: 29.07.1988 US 226303; 17.05.1989 US 353312
(43) Date of publication of application: 31.01.1990
(73) Proprietor: CIBA-GEIGY AG, 4002 Basel (CH)
(72) Inventor: Meins, Frederick, Prof., CH-4125 Riehen (CH); Shinshi, Hideaki, Tsuchinra City Ibaraki 300 (JP); Wenzler, Herman C., Texas 77840-5201 (US); Hofsteenge, Jan, Dr., CH-4153 Reinach (CH); Ryals, John, Dr., Durham, NC 27713 (US); Sperisen Christoph, CH-4056 Basle (CH)

(56) References cited:
- PROCEEDINGS OF THE NATL. ACADEMY OF SCIENCES USA, vol. 85, August 1988, Washington, DC (US); H. SHINSHI et al., pp. 5541-5545
- GENE, vol. 70, no. 1, Amsterdam (NL); M. DE LOOSE et al., pp. 13-23
- THE EMBO JOURNAL, vol. 4, no. 7, 1985, Oxford (GB); D. MOHNEN et al., pp. 1631-1635
- PROCEEDINGS OF THE NATL. ACADEMY OF SCIENCES USA, vol. 83, April 1986, Washington, DC (US); G.B. FINCHER et al., pp. 2081-2085
- PROCEEDINGS OF THE NATL. ACADEMY OF SCIENCES USA, vol. 84, July 1987, Washington, DC (US); G. BAUW et al., pp. 4806-4810
- AGRICULTURAL & BIOLOGICAL CHEMISTRY, vol. 47, no. 6, 1983, Tokyo (JP); H. SHINHI et al., pp. 1275-1280

## Description

This invention relates to DNA sequences encoding proteins which have beta-1,3-glucanase activity, or which are precursors to proteins which have beta-1,3-glucanase activity.

Glucan endo-1,3-β-glucosidases (b-1,3-glucanase) are enzymes which have been implicated in a variety of biological processes, including the defense reaction of plants against pathogens (1). They are induced in response to the stress hormone ethylene (2,3), infection (4-10) and elicitors (9,11), and have antifungal activity in vitro (12). A basic β-1,3-glucanase of tobacco of Mᵣ ∼33,000 is a major component (∼5-10 %) of the soluble protein of cultured tobacco tissues and the lower leaves and roots of tobacco plants (13,14). This b-1,3-glucanase exhibits complex hormonal and developmental regulation. It is induced in tobacco leaves treated with ethylene (3) or infected with tobacco mosaic virus (TMV) (8,10) and is down-regulated at the mRNA level in cultured cells of tobacco by combinations of the plant hormones auxin and cytokinin (3,14-16).

Glucanases are enzymes which hydrolyze polysaccharides. Accordingly they are important participants in the degradation of plant polysaccharide materials. As such, they are useful in biomass conversion (composting) processes. Under certain circumstances this activity can also be extended to paper-making processes.

The identification of the DNA sequences which encode these enzymes would lead to the ability to manipulate plants containing these genes to control expression of the corresponding proteins in those plants. In addition, removal of these DNA sequences from their natural genomes would permit transformation of other organisms with these DNA sequences in order to extend the benefits of such enzymes to organisms other than those in which the enzymes occur naturally. Furthermore, one could control the timing and extent of expression of these enzymes, thereby optimizing the value of these transformed plants. Of particular interest would be the transformation of a species of plant with genes encoding glucanases derived from another species of plant.

The present invention is directed to DNA sequences which encode polypeptides having beta-1,3-glucanase activity. Those DNA sequences may be recombinant DNA sequences, such as cDNA sequences, or they may be genomic DNA sequences recovered in substantially pure form. The DNA sequences embrace both the sequences which encode the pre-pro-enzyme as well as the mature enzyme which is formed naturally as a result of post-translation processing.

In addition, the invention embraces also DN sequences which show substantial sequence homology to such recombinant DNA sequences or genomic DNA sequences.

In a preferred embodiment the DNA sequences encode the beta-1,3-glucanases which are found naturally in plants. Of particular interest are the glucanases of tobacco.

### BRIEF DESCRIPTION OF THE FIGURES

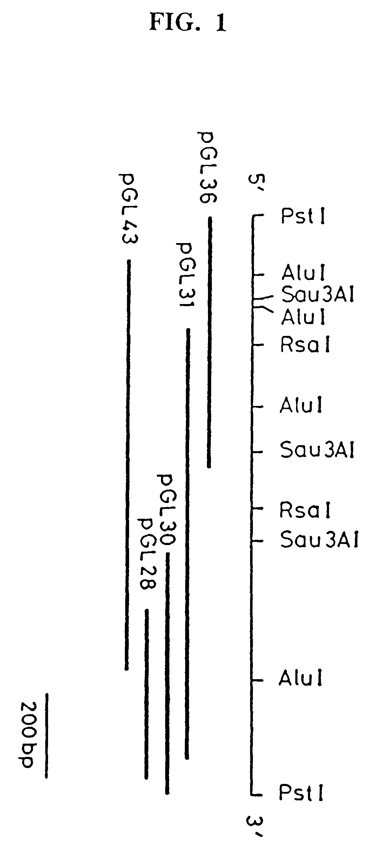

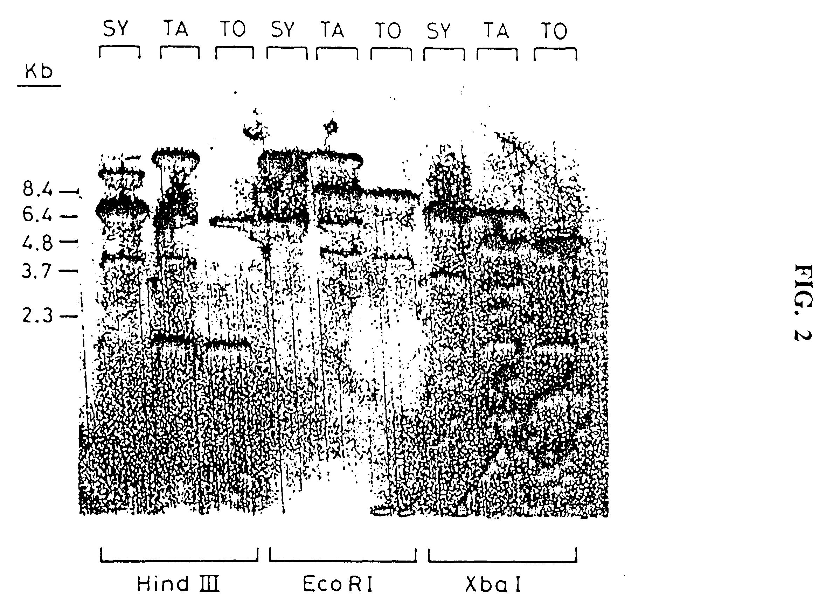

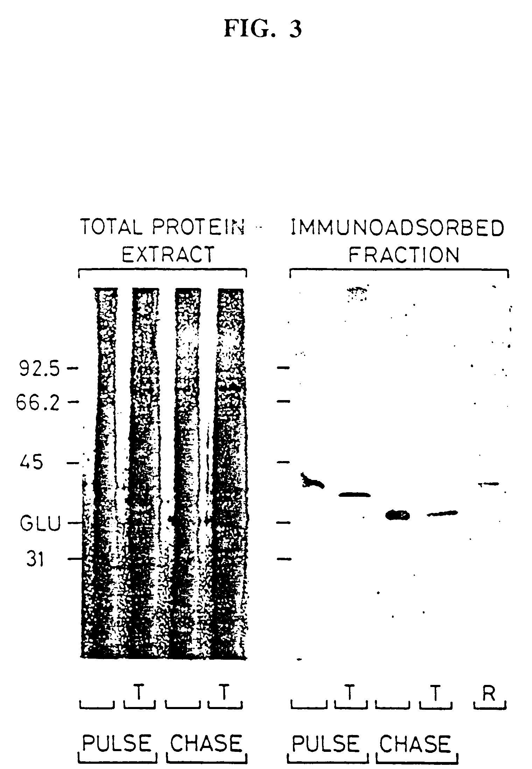

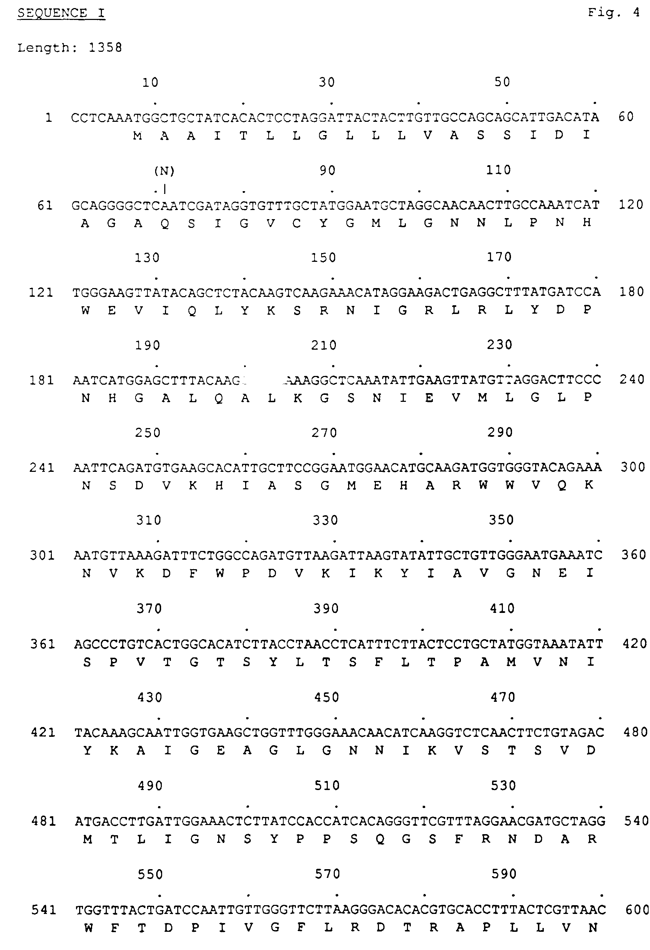

Fig. 1 provides a partial restriction map showing the lengths and positions of the cDNA clones of tobacco β-1,3-glucanase that were sequenced.

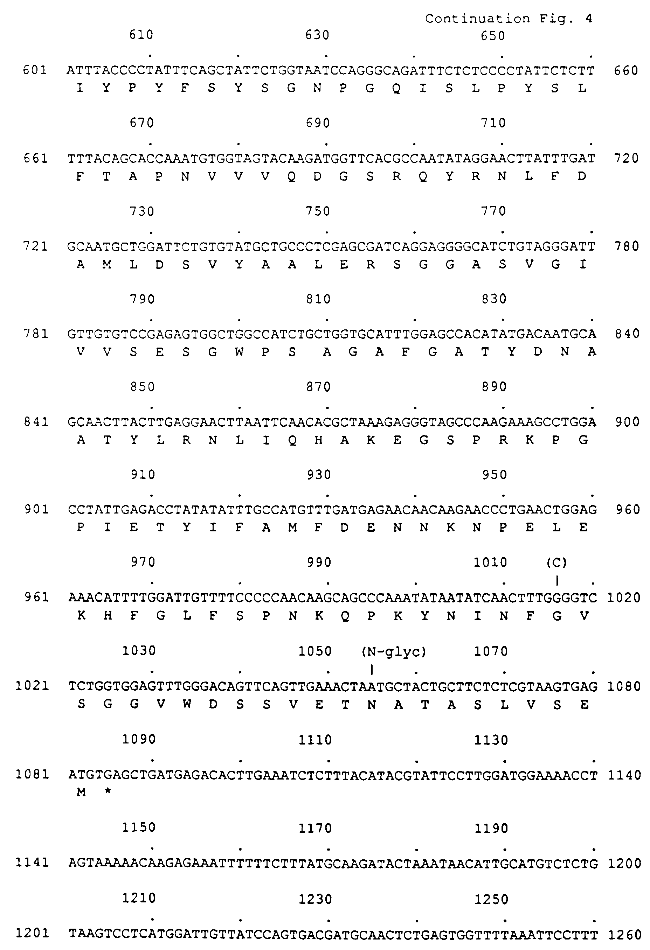

Fig. 2 presents Southern blot analysis of DNA prepared from leaves of tobacco, Nicotiana tabacum L. cv. Havana 425 (TA), N. sylvestris (SY), and N. tomentosiformis (TO) digested with the restriction endonuclease indicated and hybridized with the insert of tobacco β-1,3-glucanase cDNA plasmid pGL43. Scale at left: Size of marker DNAs in kilobases.

Fig. 3 illustrates an autoradiogram of polypeptides from tobacco protoplasts incubated for 45 min. with 35S-methionine (PULSE) and then ∼12 hr with 1 mM methionine (CHASE). Immuno-adsorption performed with anti-tobacco β-1,3-glucanase. R: Products obtained by in vitro translation of total RNA from cells induced to produce β-1,3-glucanase. T: Protoplasts treated with tunicamycin. Scale at left: Mᵣ of protein standards. GLU: Position of purified β-1,3-glucanase.

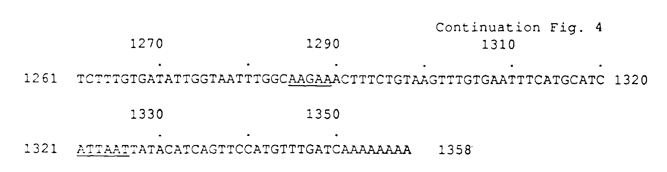

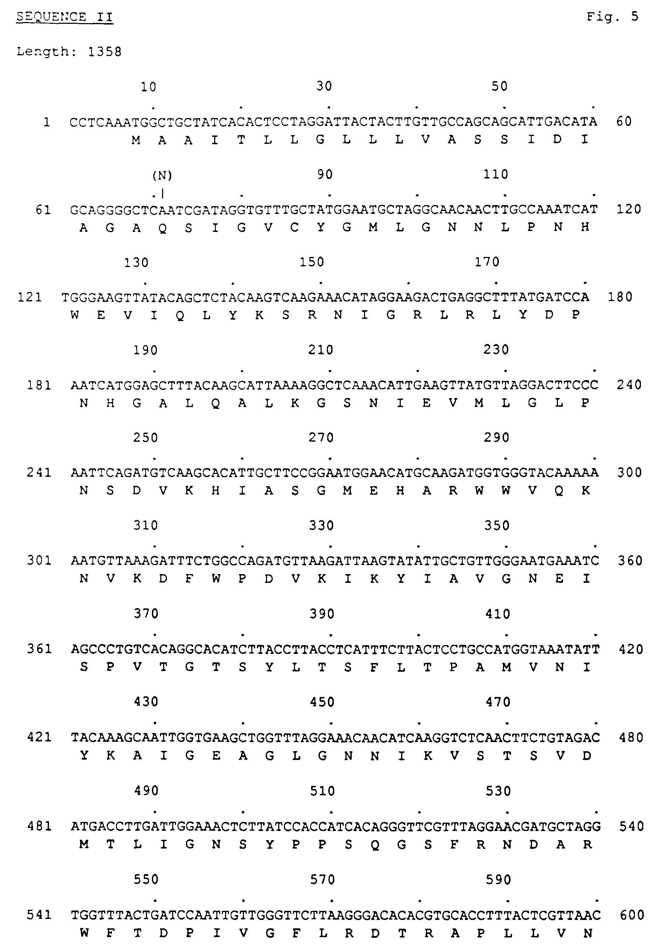

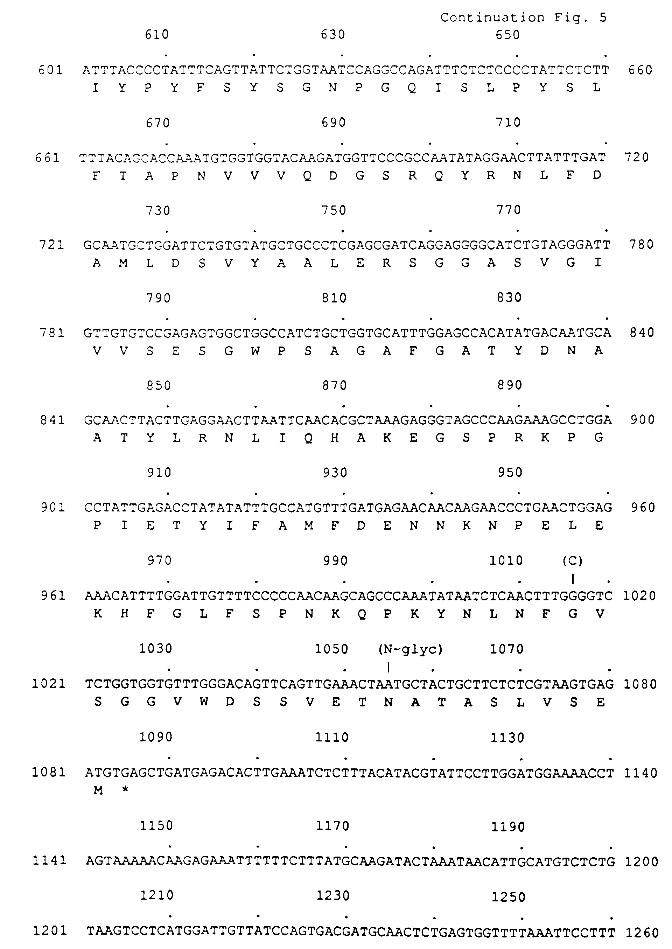

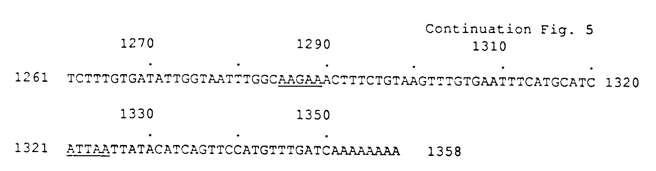

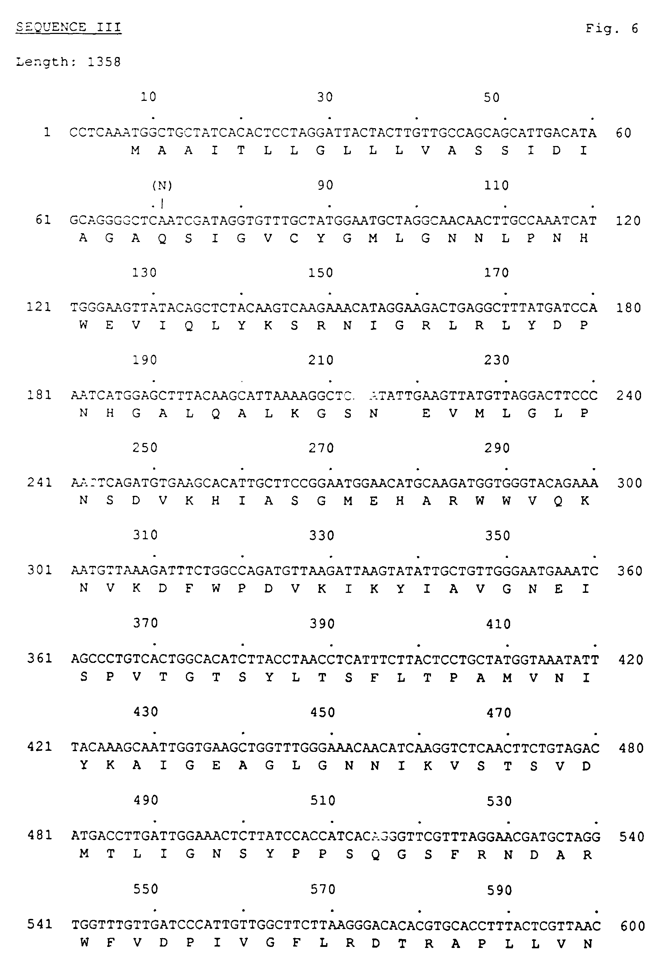

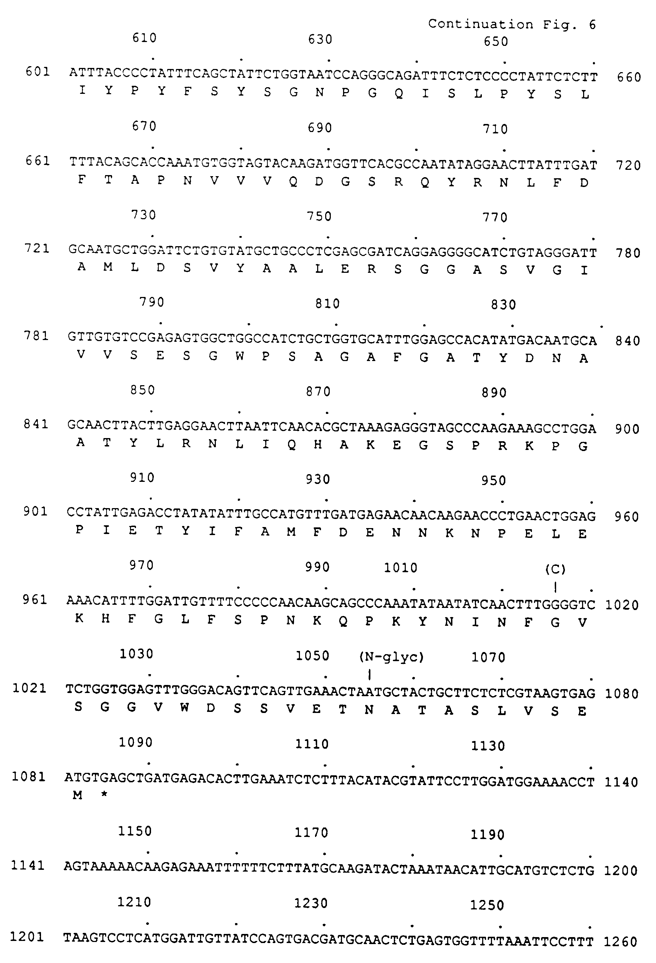

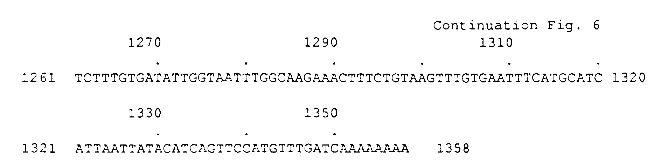

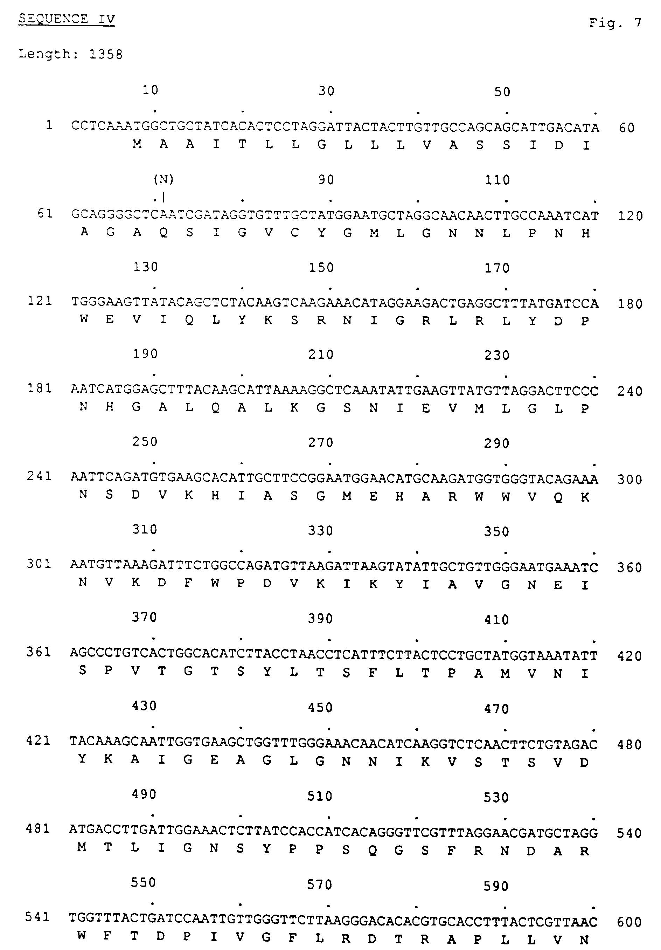

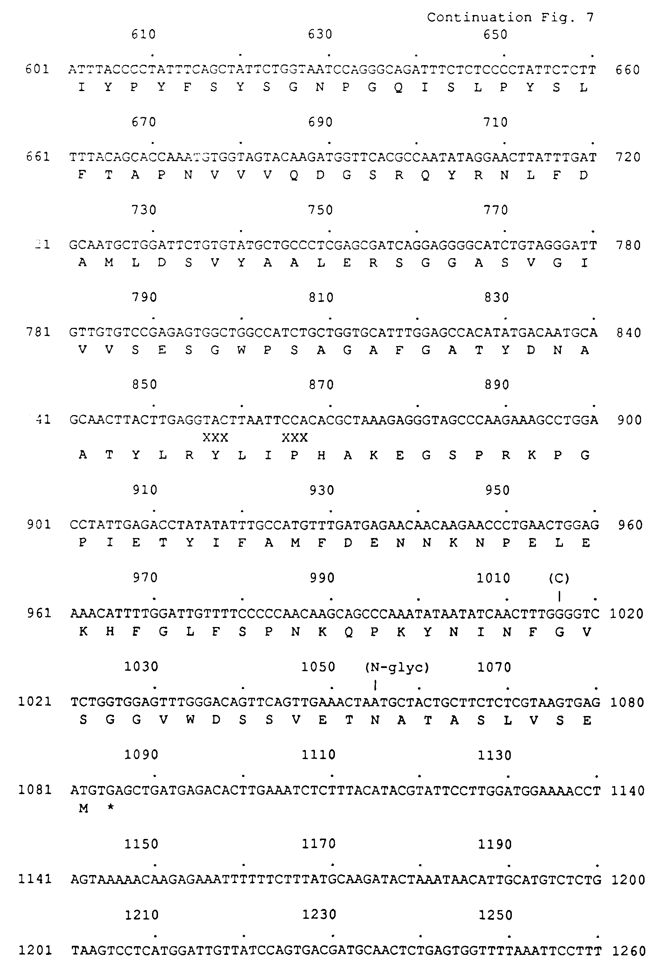

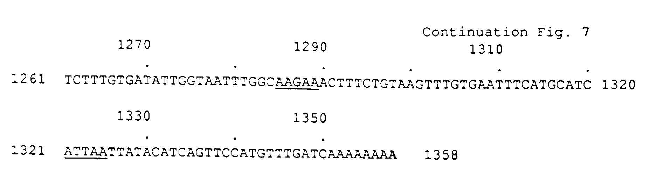

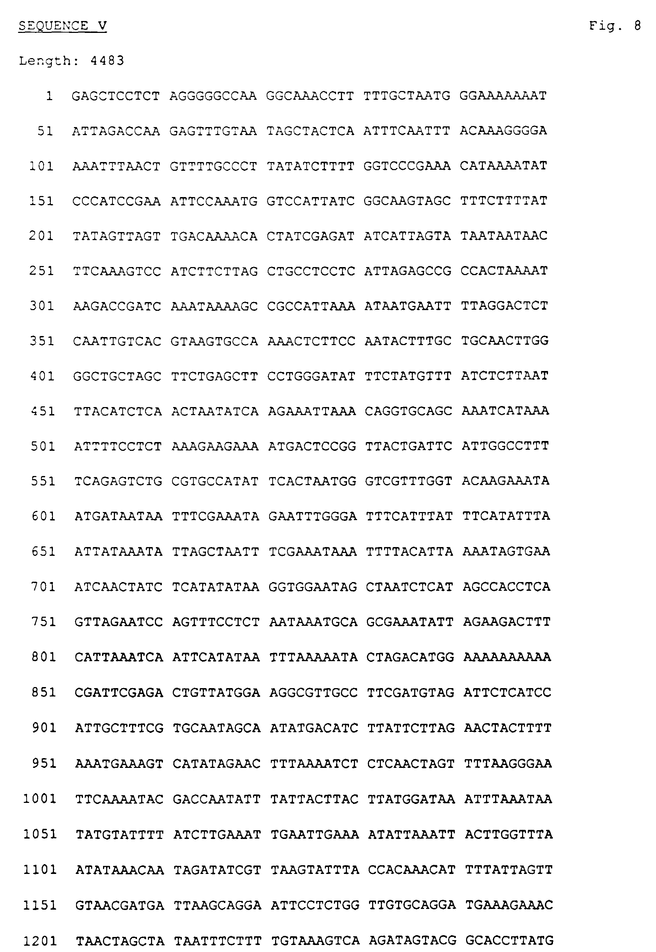

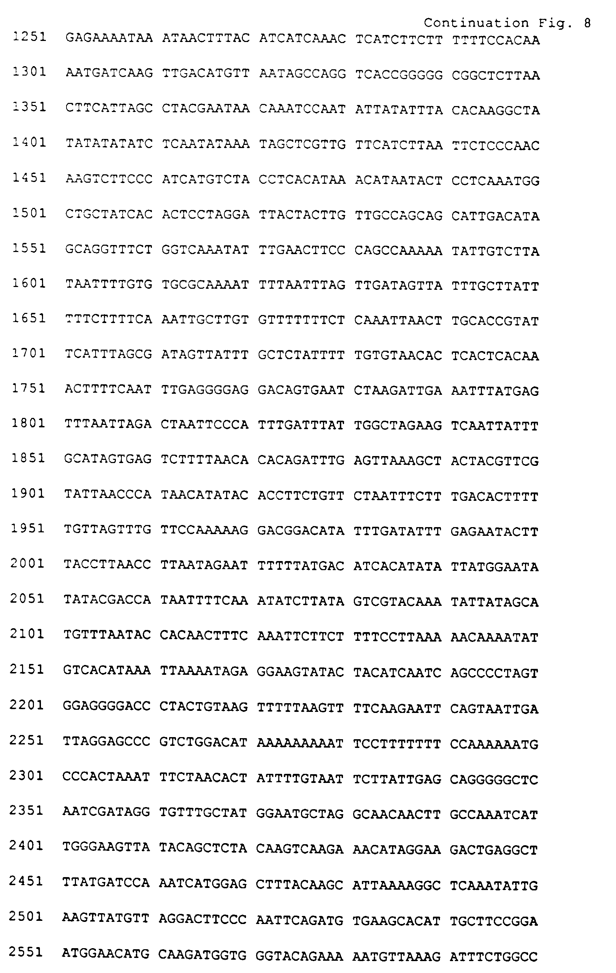

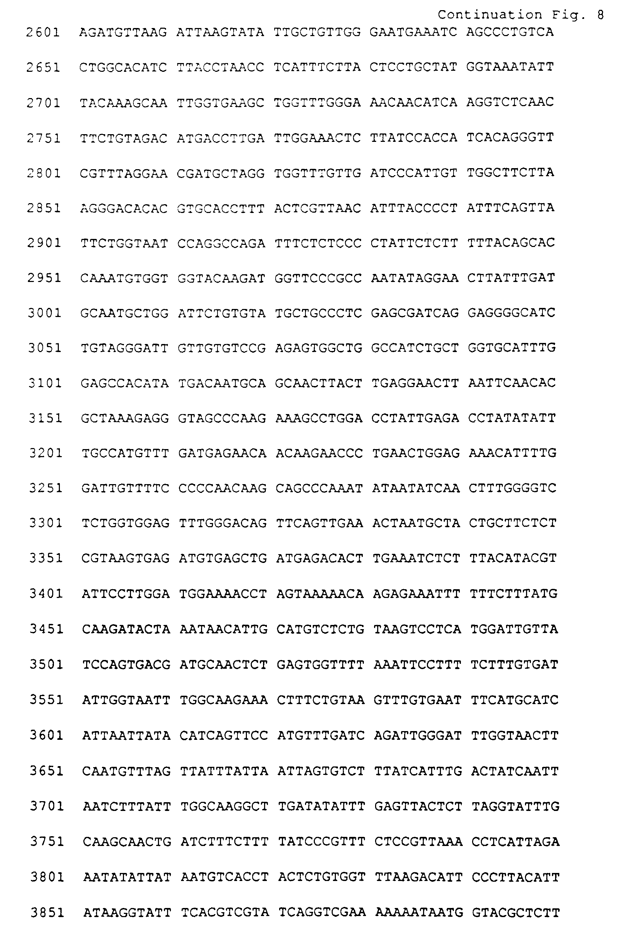

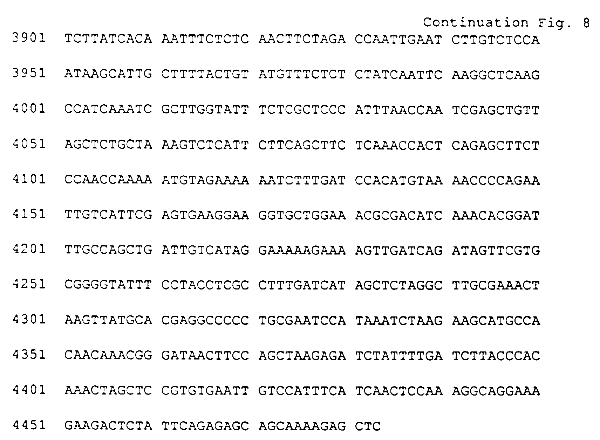

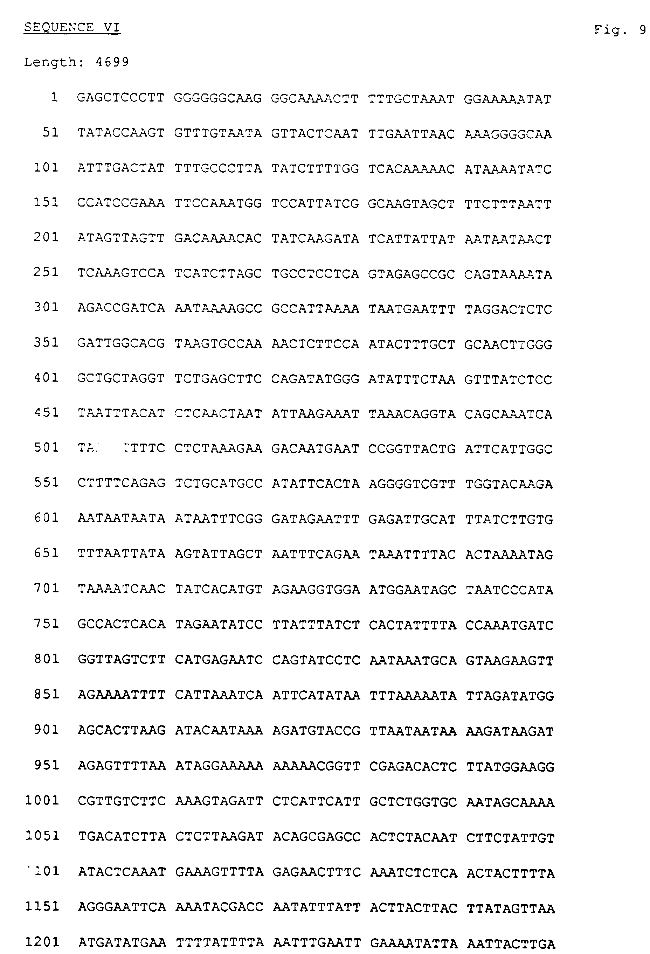

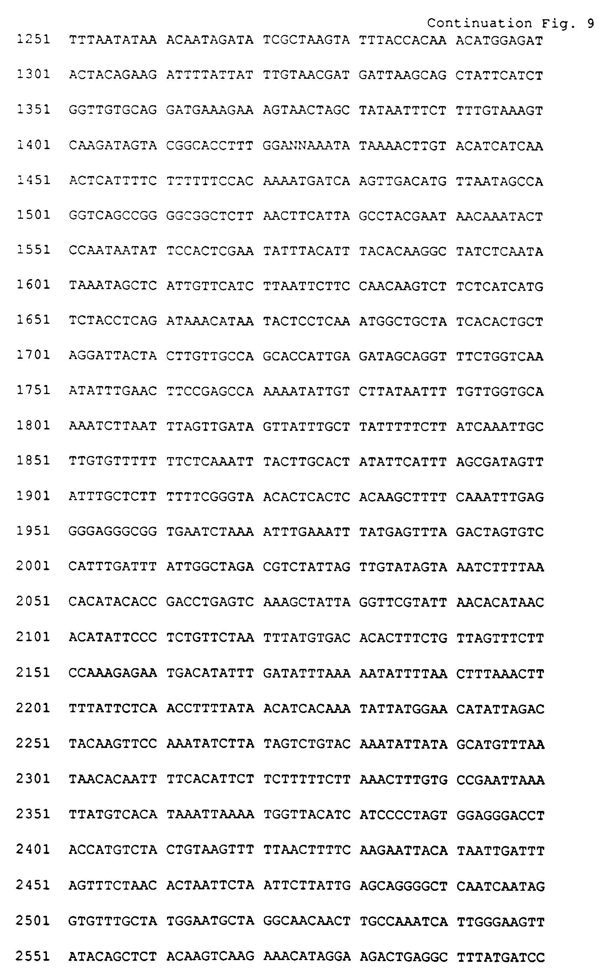

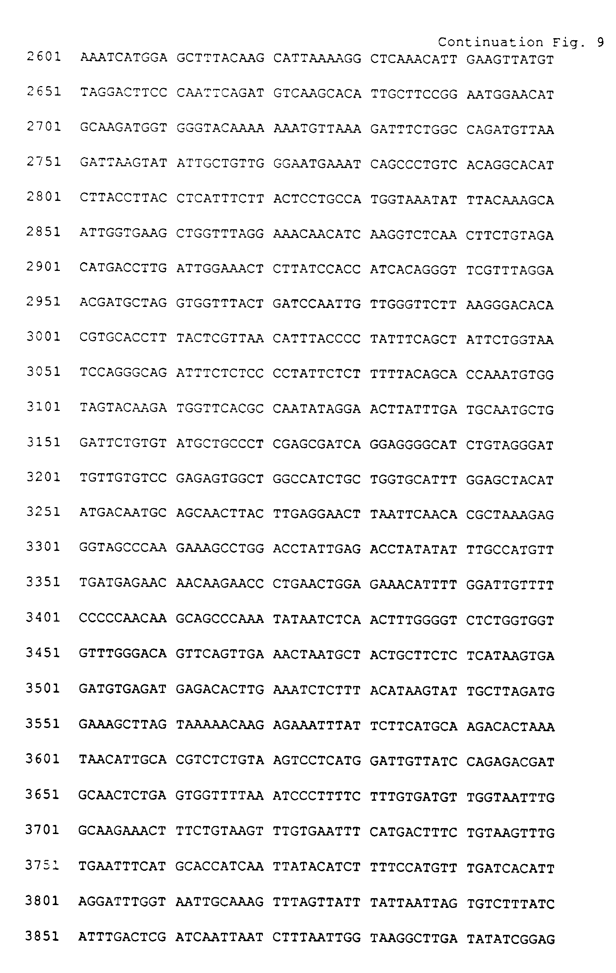

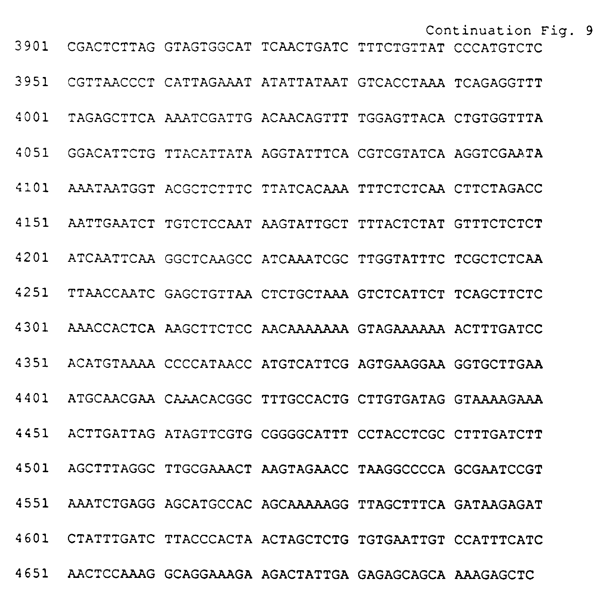

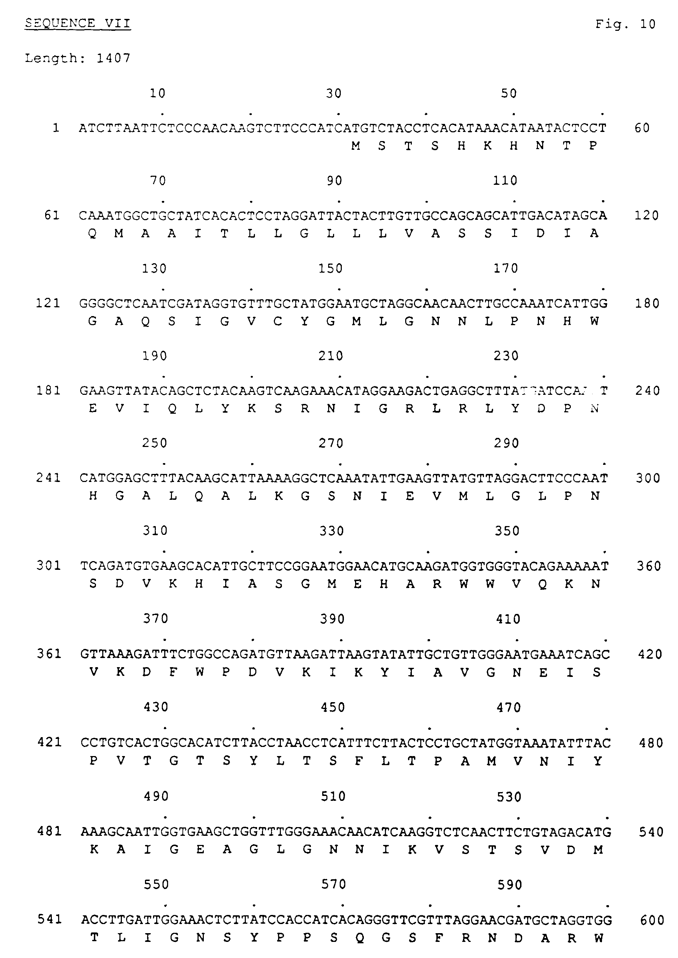

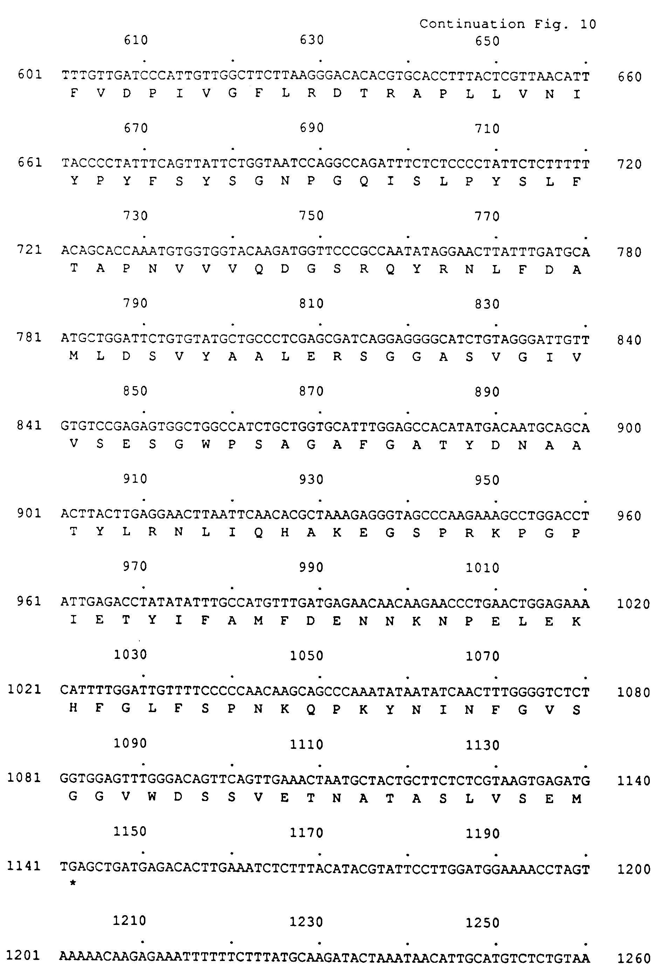

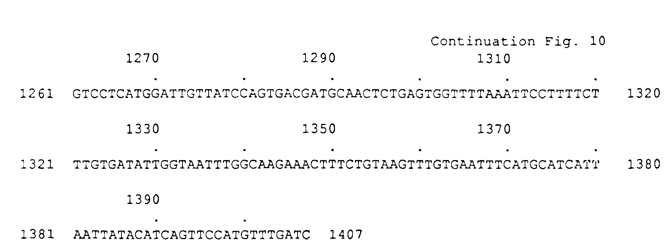

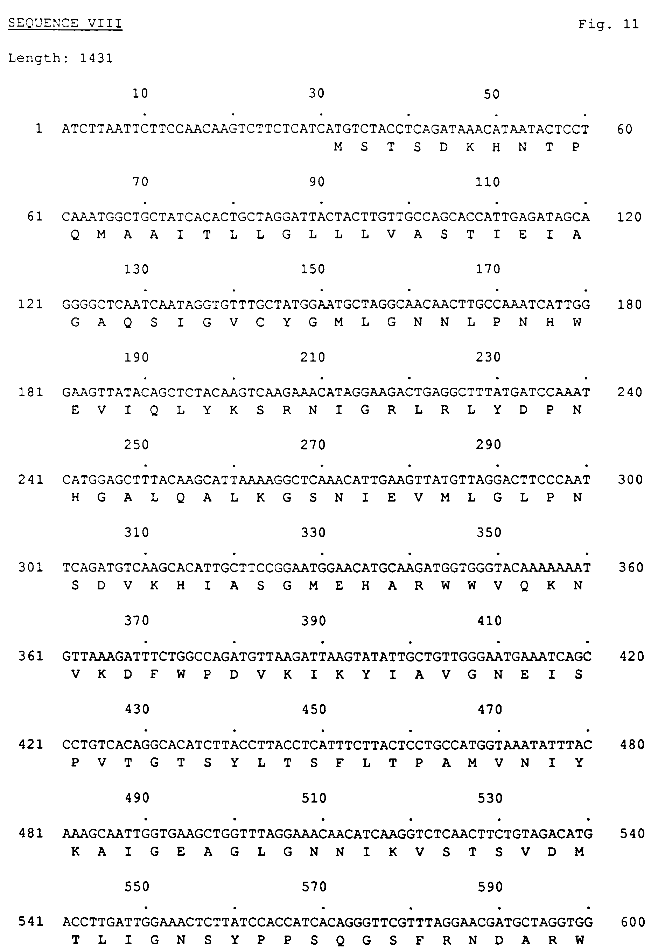

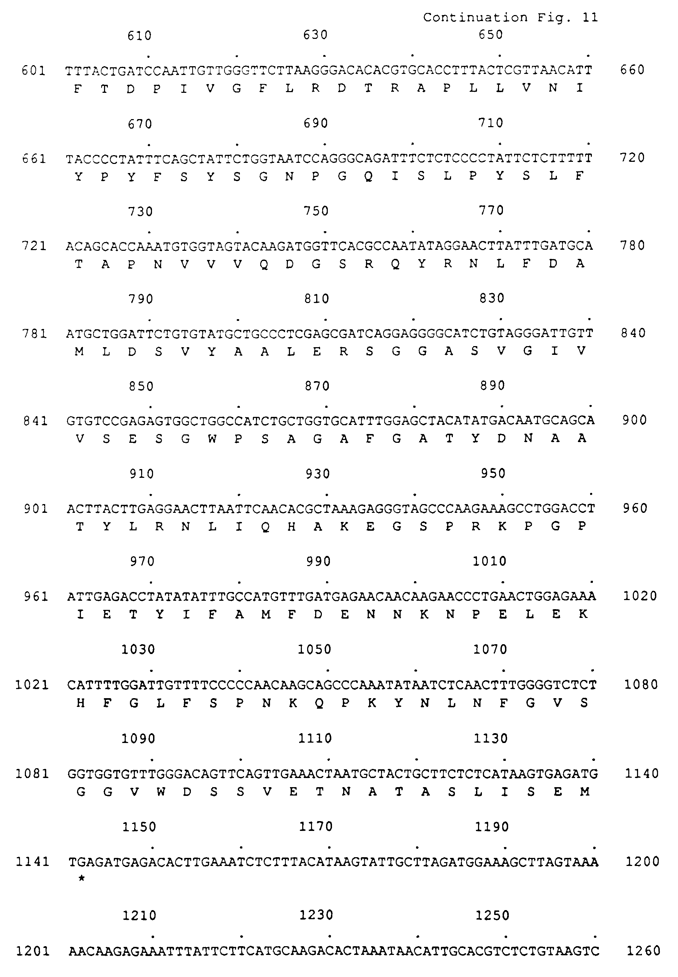

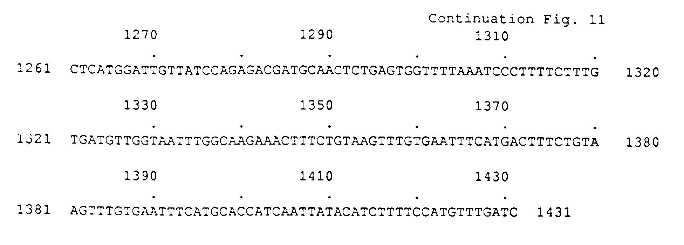

### BRIEF DESCRIPTION OF THE SEQUENCES OF FIGURES 4 TO 11

One letter symbols are used for denoting nucleotides and amino acids. (N) and (C): N- and C-terminal amino acids of mature beta-1,3-glucanase. (N-glyc): Putative N-glycosylation site. Putative polyadenylation signals are underlined.

Fig 4: SEQUENCE I: Sequence DNA-I correspond to the composite cDNA sequence from clones pGL28, pGL30 and pGL31, the sequences of which are identical. Positioned under this sequence is the deduced amino acid Sequence AA-I.

Fig 5: SEQUENCE II: Sequence DNA-II corresponds to the composite cDNA sequence derived from the clone designated pGL43. Positioned under this sequence is the deduced amino acid Sequence AA-II.

Fig 6: SEQUENCE III: Sequence DNA-III corresponds to the composite cDNA sequence derived from the clone designated pGL36. Positioned under this sequence is the deduced amino acid Sequence AA-III.

Fig 7: SEQUENCE IV: Sequence DNA-IV corresponds to the composite cDNA sequence derived from Sequence DNA-I and results of peptide sequencing shown as amino acid Sequence AA-IV, positioned under the Sequence DNA-IV. XXX indicates differences in encoded amino acids of Sequence DNA-I and those found on peptide sequencing.

Fig 8: SEQUENCE V: Sequence V corresponds to the genomic DNA as isolated in clone pBSGluc39.1.

Fig. 9: SEQUENCE VI: Sequence VI corresponds to the genomic DNA as isolated in clone pBSGluc39.3.

Fig 10: SEQUENCE VII: Sequence DNA-VII corresponds to the cDNA sequence as deduced from the isolated genomic DNA in clone pBSGluc39.1, Sequence V. Positioned under this sequence is the deduced amino acid Sequence AA-VII.

Fig 11: SEQUENCE VIII: Sequence DNA-VIII corresponds to the cDNA sequence as deduced from the isolated genomic DNA in clone pBSGluc39.3, Sequence VI. Positioned under this sequence is the deduced amino acid Sequence AA-VIII.

### DEPOSITIONS

Plasmid pBSGluc39.1 has been deposited at the American Type Culture Collection on December 29, 1988, under ATCC number 40526.

Plasmid pBSGluc39.3 has been deposited at the American Type Culture Collection on December 29, 1988, under ATCC number 40527.

### DETAILED DESCRIPTION OF THE INVENTION

The present invention is directed to a recombinant DNA sequence comprising a DNA sequence encoding a polypeptide having beta-1,3-glucanase activity, or a DNA sequence having substantial sequence homology to a DNA sequence selected from the group consisting of Sequence DNA-I, Sequence DNA-III, Sequence DNA-IV, Sequence V, Sequence VI, Sequence DNA-VII, and Sequence DNA-VIII. This recombinant DNA may comprise a molecular DNA fragment from genomic DNA encoding apolypeptide having beta-1,3-glucanase activity, or a DNA sequence having substantial sequence homology to such a fragment. Furthermore, this recombinant DNA may be a cDNAprepared from mRNA encoding a polypeptide havingbeta-1,3-glucanaseactivity or from a fragment of such mRNA, a DNA having substantial -sequence homology to such a cDNA, orarecombinant DNA comprising sucha cDNA or a DNA having substantial sequence homology to sucha cDNA. The present invention is also directed to a genomicDNA sequence encodinga polypeptide having beta-1,3-glucanase activity in substantially pure form.

Typically the source organism for the genomic DNA or the mRNA encoding a polypeptide having beta-1,3-glucanase activity will be a plant. Plants taken into consideration are monococts or dicots, for example corn, wheat, rice, barley, tobacco, tomato, cucumber, potato, French bean, soybean, pea, or carrot. Preferably the plant is tobacco.

As will be discernible from the discussion below, in tobacco the genomic DNA sequence encodes a so-called "pre-pro-enzyme" which undergoes post-translational processing to yield the mature beta-1,3-glucanase. That is, the C-terminal tail and the N-terminal tail of the pre-pro-enzyme are cleaved after translation. The sites of these cleavages are indicatedabove the nucleotide sequences in Sequences I-IV as (C) for the C-terminal cleavage point and as (N) for the N-terminal cleavage point. Accordingly, the present invention embraces the DNA sequences which encode the pre-pro-enzyme as well as the DNA sequences which encode the mature enzyme. Thislatter DNA sequences may be obtained by conventional restriction and digestion processes, either from the genomicDNA or from the cDNA encoding the pre-pro-enzyme. The invention also embraces DNA sequences having substantialsequence homology to the naturally occurring genomic DNAsequences and to cDNA sequences derived from naturally occurring mRNA sequences.

Preferred is a recombinant DNA sequence comprising a cDNA sequence encoding a mature beta-1,3-glucanase or a fragment thereof comprising the 5' end of the coding sequences, in particular such a fragment having at least 700 nucleotides or at least 900 nucleotides. Particularly preferred is a recombinant DNA sequence comprising a cDNA sequence encoding a prepro-beta-1,3-glucanase or a fragment thereof comprisingthe 5' end of the coding sequences, in particular such afragment having at least 800 nucleotides or at least 1000 nucleotides. Most preferred are such DNA sequences comprising cDNA encoding mature tobacco beta-1,3-glucanase or tobaccopre-pro-beta-1,3-glucanase, for example a recombinant DNAcomprising a DNA sequence as shown in Sequence DNA-I, Sequence DNA-III, Sequence DNA-IV, Sequence DNA-VII, or Sequence DNA-VIII. Also highly preferred is essentially puregenomic DNA encoding tobaccobeta-1,3-glucanase, for example essentially pure genomic DNA gluc39.1 (Sequence V) or genomic DNA gluc39.3 (Sequence VI), and recombinant DNA comprising said genomic DNA.

Also preferred is a recombinant DNA sequence encoding apolypeptide of the formula 
Q S I G V C Y G M L G N N L P N H W
E V I Q L Y K S R N I G R L R L Y D P N
H G A L Q A L K G S N I E V M L G L P N
S D V K H I A S G M E H A R W W V Q K N
V K D F W P D V K I K Y I A V G N E I S
 
P V T G T S Y L T S F L T P A M V N I Y
K A I G E A G L G N N I K V S T S V D M
T L I G N S Y P P S Q G S F R N D A R W
F V/T D P I V G F L R D T R A P L L V N I
Y P Y F S Y S G N P G Q I S L P Y S L F
T A P N V V V Q D G S R Q Y R N L F D A
M L D S V Y A A L E R S G G A S V G I V
V S E S G W P S A G A F G A T Y D N A A
T Y L R N/Y L I Q/P H A K E G S P R K P G P
I E T Y I F A M F D E N N K N P E L E K
H F G L F S P N K Q P K Y N I/L N F G
 in particular of the formula 
M S T S H K H N T P
Q M A A I T L L G L L L V A S S/T I D/E I A
G A Q S I G V C Y G M L G N N L P N H W
E V I Q L Y K S R N I G R L R L Y D P N
H G A L Q A L K G S N I E V M L G L P N
S D V K H I A S G M E H A R W W V Q K N
V K D F W P D V K I K Y I A V G N E I S
P V T G T S Y L T S F L T P A M V N I Y
K A I G E A G L G N N I K V S T S V D M
T L I G N S Y P P S Q G S F R N D A R W
F V/T D P I V G F L R D T R A P L L V N I
Y P Y F S Y S G N P G Q I S L P Y S L F
T A P N V V V Q D G S R Q Y R N L F D A
M L D S V Y A A L E R S G G A S V G I V
V S E S G W P S A G A F G A T Y D N A A
T Y L R N/Y L I Q/P H A K E G S P R K P G P
I E T Y I F A M F D E N N K N P E L E K
H F G L F S P N K Q P K Y N I/L N F G V S
G G V W D S S V E T N A T A S L V/I S E M
 wherein letters separated by a slash represent either amino acid. These amino acid sequences correspond to the pre-pro-enzyme and the mature enzyme, respectively, and are deduced from the amino acid sequences AA-I to IV, VII and VIII.

The invention further embraces the signal peptide sequences characteristic of the beta-1,3-glucanases. Signal peptides are amino acid components of translated proteins and enable transport of such proteins from the site of synthesis to the site of utilization. Once transport is completed, the signal peptide is cleaved, and the mature protein is rendered active or available for additional processing. In particular the invention concerns a signal peptide of tobacco beta-1,3-glucanase and recombinant DNA sequences encoding such a signal peptide, for example the peptide of the formula 
M S T S H K H N T P Q M A A I T L L G L
L L V A S S/T I D/E I A G A
 wherein letters separated by a slash represent either amino acid, and a recombinant DNA sequence encoding this peptide.

Particular DNA sequences have been prepared and characterized as cDNA or genomic sequences according to the methods set forth in the Examples. Results of the characterizations are summarized below.

Three different classes of cDNA clones are isolated with similar coding sequences. In addition, there is heterogeneity in the amino acid sequence not represented in the mRNAs corresponding to the cDNA clones isolated. Thus, cultured tobacco cells induced to produce beta-1,3-glucanase have at least four transcriptionally active beta-1,3-glucanase genes. This finding and the small number of restriction fragments detected in Southern blot experiments indicate that tobacco beta-1,3-glucanase is encoded by a small gene family. Modern tobacco arose by the hybridization of two ancestral species, N. sylvestris and N. tomentosiformis (32,33). Comparison of the restriction fragments generated from DNA of tobacco and the ancestral species suggests that ∼2-3 members of the tobacco beta-1,3-glucanase family have their evolutionary origin in each of these ancestors.

Computer searches of the EMBL version 14 (38) and NBRF version 15 (39) databanks give only one protein with significant sequence similarity to tobacco β-1,3-glucanase. After introducing 3 gaps into the sequence of the maturetobacco enzyme, the amino acid sequence of barley endosperm (1→3, 1→4)-b-glucanase isozyme II (40) is identical at 47 % of the positions. In addition, there are highly conserved regions (12 of 15 positions) at the N- and C-terminal ends of the mature proteins. The sequence of the first 22 N-terminal amino acids of a putative β-1,3-glucanase purified from N. plumbaginifolia (41) is identical to the N-terminal sequence of the tobacco enzyme except for the N-terminal amino acid, which is Glu.

Pulse chase experiments provide evidence that pre-pro-β-1,3-glucanase is processed sequentially. First, the N-terminal signal peptide is removed and an oligosaccharide side chain is added as has been reported for the seed storage proteins phaseolin and concanavalin A (42,43). Later, the oligosaccharide and the C-terminal extension are removed to give the mature enzyme. Loss of C-terminal extensions has been reported for the plant proteins, pro-thaumatin (44), pro-concanavalin A (43,45,46), and isolectin 3 of wheat germ agglutinin (47). No significant sequence similarities are found between these peptides and the C-terminal extension of the tobacco enzyme.

The cDNA sequence shown as Sequences DNA-VII is deduced from the genomic DNA sequences of clone pBSGluc3 .1, Sequence V. Nucleotides 1 to 121 correspond to nucleotides 1434 to 1554, and nucleotides 122 to 1407 correspond to nucleotides 2345 to 3630 of the genomic sequence. Nucleotides 1555 to 2344 of genomic Sequence V represent an intron. The cDNA sequence shown as Sequences DNA-VIII is deduced from the genomic DNA sequences of clone pBSGluc39.3, Sequence VI. Nucleotides 1 to 121 correspond to nucleotides 1618 to 1738, and nucleotides 122 to 1431 correspond to nucleotides 2486 to 3795. Nucleotides 1739 to 2487 of genomic Sequence VI represent an intron.

The following examples are offered to illustrate the invention as it has been developed for the tobacco plant. They are not intended to be limiting of the scope and meaning of the claims which follow.

### EXAMPLES

### Example 1: Plant Material and Culture Methods

Nicotiana tabacum L. cv. Havana 425, N. sylvestris, and N. tomentosiformis aree grown from seed in a greenhouse. The cloned line of Havana 425 pith tissue, 275N (17), is cultured under conditions that induce β-1,3-glucanase accumulation (15). Suspensions of leaf protoplasts are prepared from axenic Havana 425 shoot cultures and cultured in liquid K3 medium as described by Paszkowski et al. (18)

### Example 2: Construction and Screening of a cDNA Library

A cDNA library is prepared from poly(A⁺) RNA isolated from 275N tissue (15) as described (19) and inserted into the Pst I site of plasmid pBR322 by the homopolymeric dC-dG-tailing method (20). Recombinant colonies are screened by colony hybridization with the 32_{P}-labeled Pst I insert of tobacco β-1,3-glucanase cDNA clone pGL43, a partial cDNA clone for tobacco β-1,3-glucanase (15), as a hybridization probe. Although β-1,3-glucanase mRNA is an abundant component of the poly(A⁺) RNA used to prepare the cDNA libraries (15), only a small number of partial cDNA clones can be identified in screens of several libraries.

### Example 3: Deoxynucleotide Sequence Analysis

Restriction fragments are cloned into the M13 vectors mp18 and mp19 (21) and sequenced in both orientations by the dideoxynucleotide-chain termination method (22). Nucleotide sequences are analyzed using the computer program of Queen and Korn (23).

Five of the 7 positive recombinant clones found to have the longest cDNA inserts were sequenced (Fig. 1). Three of the clones did not include 3′ regions of the mRNA. The composite sequence shows a single, uninterrupted open reading frame of 1083 nucleotides beginning at position 1. Based on the available information of these 7 clones, the following determination of the start of the translation was made: The consensus sequence for the start of translation in plants is AACAAUGGC, in which the purine at -3 is important for efficient translation in a reticulocyte lysate system, but not in a wheat germ system (28). The first available initiation codon in the frame is at position 7, which gives the sequence UCAAAUGCG. Translation starting with this codon generates a polypeptide 359 residues long with Mᵣ 39,173 corresponding in size with the primary translation product of tobacco b-1,3-glucanase (15,29).

The overlapping sequences of the inserts of clones pGL28, pGL30 and pGL31 are identical, suggesting that they represent the same mRNA (Sequence DNA-I). Comparison of the inserts of pGL31 and pGL36, which overlap for a length of 328 nucleotides, shows two silent substitutions at positions 555 and 564 and two substitutions at positions 547 and 548 resulting in a single amino acid substitution of Thr for Val. The sequence of the pGL43 insert differs from the sequences of the pGL31 and pGL36 inserts at 13 positions; 12 are silent differences in the third wobble position of the codon and one at position 1006 results in the conservative change from Leu to Ile which is also detected by amino acid sequencing of the mature enzyme.

The composite cDNA sequence has two putative polyadenylation signals for plants, AAG AAA at position 1285 and ATT AAT at position 1321 (30). The overlapping sequences of the cDNA inserts from pGL28 and pGL30 are identical up to position 1310, where the poly(A) tail of pGL28 starts 18 nucleotides downstream from the first polyadenylation signal. The poly(A) tail of pGL30 starts 24 nucleotides downstream from the second polyadenylation signal. This suggests that there is alternate polyadenylation of the β-1,3-glucanase transcripts as reported for other plant genes (31).

### Example 4: Southern Blot Analysis

DNA (5 µg) prepared from leaves of tobacco (N. tabacum), N. sylvestris and N. tomentosiformis (32,33) by the method of Murray and Thompson (24) is digested to completion with the restriction endonucleases EcoRI, HindIII or XbaI. The resultant fragments are separated by electrophoresis on a 0.8 % (wt/vol) agarose gel, transferred to a Zeta-Probe nylon membrane (BioRad), and hybridized as recommended by the manufacturer. The final wash is performed at 50°C in 0.2 x SSC (1 x SSC is 0.15 M NaCl/15mM sodium citrate, pH 7), 0.1 % (wt/vol) sodium dodecyl sulfate (SDS). The Pst I insert of the tobacco β-1,3-glucanase cDNA clone pGL43 (15) is used as a probe and is labeled using a random primer labeling kit (Boehringer).

The restriction endonuclease fragments that hybridize to the insert of tobacco β-1,3-glucanase cDNA clone pGL43 are shown in Figure 2. Each of the restriction endonucleases generates four fragments with tobacco DNA. The EcoRI and Xbai fragments correspond in size and intensity of hybridization to two fragments generated with DNA from each of the progenitor species, N. sylvestris and N. tomentosiformis. HindIII generates fragments with N. sylvestris DNA which do not correspond in size to the fragments generated with tobacco DNA. Analyses using combinations of restriction nucleases and other hybridization probes suggest that differences in intensity of hybridization reflect differences in homology rather than copy number.

### Example 5: Analysis of Proteins

β-1,3-Glucanase is purified from 275N tissue through the CM-Sephadex G-50 chromatography step for chitinase (16). Fractions containing b-1,3-glucanase are pooled, concentrated by ultrafiltration (immersible CX-10; Millipore), adjusted to 100 mM NaCl/10 mM Tris HCl, pH 8.4, and passed over a column containing regenerated chitin (25) to remove traces of chitinase. The final product is >95 % pure as judged by SDS-PAGE and staining the protein with Coomassie blue R-250. To prepare tryptic peptides, b-1,3-glucanase (320 µg in 1 ml) is adjusted to 8 M urea and dialyzed against 2 M urea/0.1 M NH₄HCO₃. Trypsin (7 µg; Worthington) is added and the mixture incubated for 6.5 hr at 37°C. Additional trypsin (7 µg) is added and the reaction stopped after 24 hr by adjusting the pH to 3 with HCl. The procedures for CNBr cleavage, fractionation of peptides by HPLC, amino acid analysis, and amino acid sequencing are as described (26).

The sequence of 4 tryptic peptides and a CNBr peptide of the pure enzyme covering a total of 70 amino acid residues are the same as the deduced sequences, suggesting that the cloned cDNAs are derived from the mRNA for β-1,3-glucanase. Additional amino acids are found at two positions in the protein, 284 and 287, that are not represented in the cDNA clones isolated.

The amino acid and DNA sequence information is used to deduce the primary structure of pre-pro-β-1,3-glucanase. Using the rules for protein processing of Von Heijne 34), a highly probable cleavage site is identified between Ala 21 and Gln 22. Processing at this site is consistent with the observation that the N-terminal end of mature b-1,3 glucanase is blocked. N-terminal glutamines are often blocked by spontaneous or enzymatic conversion to a pyroglutamyl residue (35). A purified N-terminally blocked peptide obtained by CNBr cleavage of the mature enzyme with the completion Glu_{0.6} Ser_{1.0} Ile_{1.0} Gly_{2.0} Val_{1.1} Tyr_{1.0} Hse_{1.0} (Hse is homoserine) corresponds to the N-terminal sequence Gln Ser Ile Gly Val Cys Tyr Gly Met. These results indicate that the primary translation product for β-1,3-glucanase has a hydrophobic signal peptide 21 residues in length and that the N-terminal end of the mature enzyme is Gln 22.

The primary translation product of β-1,3-glucanase has a Mᵣ ∼4,000 larger than that of the mature enzyme (15,29). The presence of a signal peptide can only account for a Mᵣ difference of ∼2,000, suggesting the protein under goes additional processing. Cleavage of the enzyme with CNBr yields a peptide that lacks homoserine indicating that it is the C-terminal end of the mature protein. Its amino acid composition and sequence were determined, and the results show it to correspond to residues 308 to 337 of the deduced sequences. Since the stop codon occurs 22 amino acid residues downstream of Gly 337, it appears that a C-terminal extension is removed during processing.

### Example 6: Pulse-Chase Experiments

Leaf protoplasts (5 x 10⁴) are incubated for 45 min at 26°C in the dark with shaking (50 rpm) in wells of a Falcon 24-well tissue culture plate (Becton Dickinson) containing 500 µl of K3 medium without phytohormones and ∼20 µCi of 35_{S}-L-methionine (specific activity ∼1275 Ci/nmol; Amersham).

Where indicated, the pulse labeling is followed by incubation for 11.75 hr with 1 mM L-methionine added to the wells. A replicate set of wells is supplemented with 20 µg/ml of tunicamycin (Calbiochem) added 20 min before the 35_{S}-L-methionine. Protein extracts are prepared bv adding 100 µl of 6 mM dithiothreitol, 6 % (vol/vol) Triton X-100, 200 mM Tris HCl, pH 8.0 to each well, incubating for 10 min at 26°C and freezing the samples in liquid N2. The extracts are divided into two aliquots. The protein in one aliquot is precipitated with acetone and analyzed by SDS-PAGE (17). The second aliquot is immunoadsorbed with rabbit anti-tobacco β-1,3-glucanase IgG (27) bound to protein A-Sepharose CL-4B (Pharmacia) and the immunoadsorbed polypeptides are analyzed by SDS-PAGE (15). 35_{S}-methionine labeled in vitro translation products are obtained with a rabbit reticulocyte system. Total RNA from 275N tissues induced to produce β-1,3-glucanase is the source of mRNA (15). Gels are calibrated with SDS-PAGE Standards, low molecular weight (BioRad).

The processing of β-1,3-glucanase deduced from structural studies (Example 5) is confirmed in pulse-chase experiments. The major polypeptide labeled following pulse labeling of tobacco protoplasts with 35_{S}-methionine for 45 min is immunoadsorbed by anti-b-1,3-glucanase antibody (Figure 3). It has a Mᵣ slightly larger than the in vitro translation product included on the same SDS-PAGE as a standard for the pre-pro-enzyme. After the chase, the majority of the immunoreactive material co-migrates with the mature form of β-1,3-glucanase, indicating that most of the precursor form of β-1,3-glucanase is processed to the mature form during the ∼12 hr chase.

The deduced sequence of the precursor has a single putative site for N-glycosylation, Asn X Ser/Thr (36), starting at Asn 350 in the C-terminal extension. This suggests that processing of β-1,3-glucanase involves glycosylation. To test this hypothesis, replicate protoplast suspensions used in the pulse-chase experiments are incubated with tunicamycin, which inhibits the synthesis of lipid-linked oligosaccharides and is known to block N-glycosylation of plant polypeptides (37). Tunicamycin treatment inhibits 35_{S}-methionine incorporation into polypeptides by ∼50 %. The immunoreactive polypeptide obtained after pulse labeling in the presence of tunicamycin has a Mr ∼2,000 smaller than the polypeptide obtained with untreated protoplasts. On the other hand, the immunoreactive polypeptide obtained following the chase treatment in the presence of tunicamycin has the same Mᵣ as mature β-1,3-glucanase. Plant N-glycans (37) and the N-terminal signal sequence of β-1,3-glucanase have the same Mᵣ, ∼2,000. Therefore, the results indicate that the precursor loses the signal peptide and gains an oligosaccharide sidechain; this occurs at the site marked (N-glyc) in the Sequences. Later, the resultant pro-enzyme is deglycosylated and the C-terminal extension is lost to give the mature enzyme. Processing to the mature form does not, however, require glycosylation.

### Example 7: Isolation of a Genomic Clone

High molecular weight DNA is prepared from leaves of Nicotiana tabacum cv. Havana 425 by the CETAB procedure (24). 100 µg of DNA is digested to completion wit SacI. This DNA is separated by electrophoresis on a 0.8 % agarose gel. The region of the gel corresponding to a molecular weight range of 3 to 7 kb is cut into 6 equally sized strips and the DNA is electroeluted from these strips and precipitated as separate fractions. The DNA is resuspended and an aliquot from each fraction is run on an agarose gel and analyzed by Southern blotting. The fraction that contains DNA which hybridizes to the β-1,3-glucanase cDNA probe (Example 4) is pooled and used in constructing libraries.

The cloning vector lambdaOngC purchased from Stratagene Corp. is digested with SacI. 1 µg of this DNA is mixed with 0.1 µg of the SacI digested tobacco DNA, and the DNA is ligated with T4 DNA ligase according to the manufacturer's suggestions. The ligated DNA is then packaged into phage particles using an in vitro packaging procedure according to Stratagene. The phages are plated with bacteria as suggested in the lambda manual. About 75,000 plaques are screened using a 32-P labeled β-1,3-glucanase cDNA probe. 11 positive plaques are identified. These plaques are purified by successive rounds of plating and screening.

DNA is isolated from the purified clones and the clones are analyzed by restriction digestion using HindIII, EcoRI, and SacI. The clones are of two types, one represented by the clone 39.1 and the other represented by the clone 39.3. The 4.5 and 4.7 kb inserts in clone 39.1 and 39.3, respectively, are subcloned into the bluescript plasmid digested with SacI, and the subclones pBSGluc39.1 (SacI fragment derived from the lambda clone 39.1) and pBSGluc39.3 (SacI fragment derived from the lambda clone 39.3) are isolated. The sequence of the DNA in the SacI fragments contained in the subclones pBSGluc39.1 and pBSGluc39.3 is determined by DNA sequencing and shown in Sequence V and VI, respectively. The coding sequence is determined and the corresponding amino acid sequence deduced. These sequences are shown in Sequence VII and Sequence VIII, repectively. A large intervening sequence is located near the 5′ end of the coding sequence.

Plasmid pBSGluc39.1 has been deposited at the American Type Culture Collection on December 29, 1988, under ATCC number 40526.

Plasmid pBSGluc39.3 has been deposited at the American Type Culture Collection on December 29, 1988, under ATCC number 40527.

### Example 8: Identification of the Transcriptional Start Site

### A. Primer Extension Mapping

A synthetic DNA primer is synthesized which is complementary to bases 2399 to 2416 (Sequence V) using an Applied Biosystems Synthesizer and β-cyanoethylphosphoramidate chemistry. The oligonucleotide is purified by reverse-phase high pressure liquid chromatography (HPLC). 5 pmol of the oligo are kinased (20) using 200 µCi of 32P-ATP (6000 Ci/mmol, 10 µCi/µl). After incubation at 37°C for 30 min, the reaction is diluted to 100 µl, extracted with phenol/chloroform and then precipitated three times with 50 µg carrier RNA. The final precipitate is resuspended in 1 X reverse-transcriptase buffer (50 mM Tris-HCl, pH 7.5, 40 mM KCl, 3 mM MgCl₂) at a concentration of 2 nM. The specific activity of the labeled oligonucleotide is determined to be about 3 X 10⁶ Cvcpm/pmol.

Total RNA is isolated from Phytophthora parasitica var. nicotiana infected tobacco essentially as described (48). Tissue is ground under liquid nitrogen in a mortar and pestle. The ground tissue is added to grinding buffer (48) using 2.5 ml per gram tissue. An equal volume of phenol is then added and the emulsion is homogenized in a Brinkman polytron. A one-half volume of chloroform is added and the emulsion is gently mixed for 15 minutes. The phases are separated by centrifugation and the aqueous phase is removed. RNA is precipitated by the addition of sodium acetate to 0.3 M and 2.5 volumes ethanol. The precipitate is collected by centrifugation and resuspended in 2 ml sterile water. Lithium chloride is added to a final concentration of 3 M and left at 4°C overnight. The precipitate is collected by centrifugation and the pellet is washed with ice-cold 80 % ethanol. The pellet is dried and resuspended in 500 µl sterile water. The concentration of this total RNA preparation is determined spectrophotometrically.

50 µg of total RNA is lyophilized in a 500 µl Eppendorf tube. The RNA is resuspended in 30 µl of radiolabeled probe solution and heated to 70°C for 10 min. The tube is slowly cooled to 37°C and allowed to incubate overnight. Without removing the tube from the 37°C water bath, 2 µl of 10 X reverse-transcriptase buffer (500 mM Tris-HCl, pH 7.5, 400 mM KCl, 30 mM MgCl₂), 1 µl 5 mg/ml bovine serum albumin, 5 µl 100 mM dithiothreitol, 5 µl 10 X dNTPs (10 mM of each dNTP in H₂O), 3 µl H₂O, 2 µl RNasin (80 units), and 2 µl reverse transcriptase (400 units) are added and the reaction is incubated at 37°C for 30 minutes. To stop the reaction, 5 µl of 3 M sodium acetate, pH 5, and 150 µl absolute ethanol are added. The tube is left at -20 C for 30 minutes, the precipitate is collected by centrifugation, washed with 80 % ethanol and allowed to air-dry. The precipitate is resuspended in 10-20 µl of loading dye (90 % formamide, 0.05 % bromophenol blue, 0.05 % xylene cyanol, 1 mM EDTA) and the extension product are separated on a 6% sequencing gel (20). Extension products are visualized by autoradiography.

The primer extension products are run against a molecular weight standard in which the labeled primer is used in dideoxy DNA sequencing reactions with the subclone pBSGluc39.1 as a template. Extension products are identified that correspond to positions 1432, 1446 and 1447 of the β-1,3-glucanase 39.1 sequence, Sequence V. Since a large intron exists between positions 1554 and 2345 of the pBSGluc39.1 sequence, the molecular weight ladder might not reflect the correct molecular weight of the extension products. Therefore, a second primer extension mapping experiment is conducted using a primer that is complementrary to positions 1530 to 1547. Using this primer, three 5′ ends of the glucanase mRNA are mapped to adenine residues at positions 1434, 1448 and 1449.

### B. S1 nuclease mapping

A synthetic oligonucleotide complementary to positions 1415 to 1504 is synthesized for use as a probe in S1 nuclease mapping. The oligonucleotide is kinased at the 5′ end using 32P-ATP and T4 polynucleotide kinase according to the supplier's recommendation. Following phenol extraction and ethanol precipitation the labeled oligonucleotide is resuspended in formamide hybridization buffer (49) at a concentration of about 2 nM. Lyophilized, total RNA (50 µg) from Phytophthora parasitica infected tobacco as above is dissolved in 30 µl of the probe solution, and the tubes are heated to 65°C for 10 minutes, then allowed to hybridize overnight at 48°C. S1 nuclease treatment and gel electrophoresis are essentially as described, using an S1 concentration of 400 units/ml and an incubation temperature of 30°C. The appropriate S1 nuclease concentration is determined in pilot experiments. After gel electrophoresis three bands are detected that correspond to positions 1434, 1448 and 1449 on the pBSGluc39.1 DNA sequence.

### C. Determination of the transcriptional start site

Primer extension and S1 nuclease mapping procedures both place the 5′ ends of the mRNA at positions 1434, 1448 and 1449. Therefore these sites, which are all adenine residues, correspond to the transcriptional start site of the β-1,3-glucanase gene.

### D. Determination of the translational start site

By comparing the composite cDNA sequences to the sequence of either genomic clone it is clear that there is an in-frame methionine initiator codon located 32 amino acids upstream of the amino terminal glutamine in the mature protein. It is concluded that this codon represents the translational start site because it is the first methionine codon after the transcriptional start site and because the resulting signal peptide is of the appropriate length (25-32 amino acids) and contains a second methionine 11 residues downstream of the frist, which is common in signal peptides.

### REFERENCES

1. Boller, T. (1987) in Plant-Microbe Interactions, Molecular and Genetic Perspectives, eds Kosuga, T. & Nester, E.W. (Macmillan, New York), Vol 2, pp. 385-413.
2. Abeles, F.B., Bosshart, R.P., Forrence, L.E. & Habig, W.H. (1971) Plant Physiol. 47, 129-134.
3. Felix, G. & Meins, F., Jr. (1987) Planta 172, 386-392.
4. Moore, A.E. & Stone, B.A. (1972) Virology 50, 791-798.
5. Pegg, G.F. & Young, D.H. (1981) Physiol. Plant Pathol. 19, 371-382.
6. Kearney, C.M. & Wu, J.H. (1984) Can. J. Bot. 62, 1984-1988.
7. Sanada, M., Matsushita, K., Shimokawa, H. & Itoh, R. (1986) Ann. Phytopath. Soc. Japan 52, 320-329.
8. Kauffmann, S., Legrand, M., Geoffroy, P. & Fritig, B. (1987) EMBO J. 6, 3209-3212.
9. Kombrink, E., Schroeder, M. & Hahlbrock, K. (1988) Proc. Natl. Acad. Sci. USA 85, 782-786.
10. Voegeli-Lange, R., Hansen-Gehri, A., Boller, T. & Meins, F., Jr. (1988) Plant Science, 54, 171-176.
11. Mauch, F., Hadwiger, L.A. & Boller, T. (1984) Plant Physiol. 76, 607-611.
12. Mauch, F. (1985) Ph.D. Dissertation, Univ. of Basel, Basel.
13. Shinshi, H. & Kato, K. (1983) Agric. Biol. Chem. 47, 1455-1460.
14. Felix, G. & Meins, F., Jr. (1986) Planta, 167, 206-211.
15. Mohnen, D., Shinshi, H., Felix, G. & Meins, F., Jr. (1985) EMBO J. 4, 1631-1635.
16. Shinshi, H., Mohnen, D. & Meins, F., Jr. (1987) Proc. Natl. Acad. Sci. USA 84, 89-93.
17. Eicholz, R., Harper, J., Felix, G. & Meins, F., Jr. (1983) Planta 158, 410-415.
18. Paszkowski, J., Shillito, R.D., Saul, M., Mandák, V., Hohn, T., Hohn, B. & Potrykus, I. (1984) EMBO J. 3, 2717-1722.
19. Gubler, U. & Hoffman, B.J. (1980) Gene 25, 263-269.
20. Maniatis, T., Fritsch, E.F. & Sambrook, J. (1982) Molecular Cloning: A Laboratory Manual (Cold Spring Harbor Laboratory, Cold Spring Harbor, NY).
21. Yanisch-Perron, C., Vieira, J. & Messing, J. (1985) Gene, 33, 103-109.
22. Sanger, F., Nicklen, S. & Coulson, A.R. (1977) Proc. Natl. Acad. Sci. USA, 74, 5463-5467.
23. Queen, C. & Korn, L.J. (1984) Nucleic Acid Res. 12, 581-599.
24. Murray, M.G. & Thompson, W.F. (1980) Nucleic Acid Res. 8, 4321-4325.
25. Boller, T., Gehri, A., Mauch, F. & Voegeli, U. (1983) Planta, 157, 22-31.
26. Hofsteenge, J. & Stone, S.R. (1987) Eur. J. Biochem. 160, 49-56.
27. Felix, G. & Meins, F., Jr. (1985) Planta, 164, 423-428.
28. Luetcke, H.A., Chow, K.C., Moss, K.A., Kern, H.F. & Scheele, G.A. (1987) EMBO J. 6, 43-48.
29. Shinshi, H. & Kato, K. (1983) Agric. Biol. Chem. 47, 1275-1280.
30. Dean, D., Tamaki, S., Dunsmuir, P., Favreau, M., Katayama, C., Dooner, H. & Bedbrook, J. (1986) Nucleic Acids Res. 14, 2229-2240.
31. Heidecker, G. & Messing, J. (1986) Ann. Rev. Plant Physiol. 37, 439-466.
32. Gray, J.C., Kung, S.D., Wildman, S.G. & Sheen, S.J. (1974) Nature, 252, 226-227.
33. Jamet, E., Durr, A. & Fleck, J. (1987) Gene, 59, 213-221.
34. Von Heijne, G. (1983) Eur. J. Biochem. 133, 17-21.
35. Abraham, G.N. & Podel, D.N. (1981) Mol. Cell. Biochem. 38, 181-190.
36. Kornfeld, R. & Kornfeld, S. (1985) Ann. Rev. Biochem. 54, 631-664.
37. Chrispeels, M.J. (1984) Philos. Trans. R. Soc. Lond. Ser. B, 304, 309-322.
38. Hamm, G.H. & Cameron, G.N. (1986) Nucl. Acid Res. 14, 5-10.
39. George, D.G., Barker, W.C. & Hunt, L.T. (1986) Nucl. Acid Res. 14, 11-16.
40. Fincher, G.B., Lock, P.A., Morgan, M.M., Lingelbach, K., Wettenhall, R.E.H., Mercer, J.F.B., Brandt, A. & Thomsen, K.K. (1986) Proc. Natl. Acad. Sci. USA, 3, 2081-2085.
41. Bauw, G., De Loose, M., Inzé, D., Van Montague, M. & Vandekerckhove, J. (1987) Proc. Natl. Acad. Sci USA, 84, 4806-4810.
42. Bollini, R., Vitale, A. & Chrispeels, M. . (1983) J. Cell. Biol. 96, 999-1007.
43. Herman, E.M., Shannon, L.M. & Chrispeels, M.J. (1985) Planta, 165, 23-29.
44. Edens, L., Heslinga, L., Klok, R., Ledeboer, A.M., Maat, J., Toonen, M.Y., Visser, C. & Verrips, C.T. (1982) Gene, 18, 1-12.
45. Bowles, D.J., Marcus, S.E., Pappin, D.J.C., Findlay, J.B.C., Eliopoulos, E., Maycox, P.R. & Burgess, J. (1986) J. Cell Biol. 102, 1284-1297.
46. Chrispeels, M.J., Hartl, P.M., Sturm, A. & Faye, L. (1986) J. Biol. Chem. 261, 10021-10024.
47. Raikhel, N.V. & Wilkins, T.A. (1987) Proc. Natl. Acad. Sci. USA, 84, 6745-6749.
48. Lagramini, L.M. et al. (1987) Proc. Natl. Acad. Sci. USA, 84, 7542.
49. Berk, A.J. et al. (1977) Cell, 12, 721.

## Claims

1. A recombinant DNA sequence comprising a DNA sequence encoding a polypeptide having beta-1,3-glucanase activity which has substantial sequence homology to a DNA sequence selected from the group consisting of Sequence DNA-I, Sequence DNA-III, Sequence DNA-IV, Sequence V, Sequence VI, Sequence DNA-VII, and Sequence DNA-VIII.

2. A recombinant DNA sequence of claim 1 wherein said DNA sequence encoding a polypeptide having beta-1,3-glucanase activity is a molecular fragment from genomic DNA.

3. A recombinant DNA sequence of claim 1 wherein said DNA sequence encoding a polypeptide having beta-1,3-glucanase activity is a cDNA prepared from mRNA or from a mRNA fragment, or a DNA sequence having substantial sequence homology to such a cDNA.

4. A recombinant DNA sequence of claim 1 which is a cDNA prepared from mRNA or from a mRNA fragment, or a DNA sequence having substantial sequence homology to such a cDNA.

5. A recombinant DNA sequence of claim 1 encoding tobacco beta-1,3-glucanase.

6. A purified genomic DNA sequence encoding a polypeptide having beta-1,3-glucanase activity which has substantial sequence homology to a DNA sequence selected from the group consisting of Sequence V and Sequence VI.

7. A purified genomic DNA sequence of claim 6 wherein said beta-1,3-glucanase is tobacco beta-1,3-glucanase.

8. A recombinant DNA sequence of claim 1 comprising a cDNA sequence encoding mature beta-1 ,3-glucanase.

9. A recombinant DNA sequence of claim 1 comprising a 5' end coding sequence fragment of a cDNA sequence encoding mature beta-1,3-glucanase.

10. A recombinant DNA sequence of claim 9 wherein the coding sequence fragment comprises at least 700 nucleotides.

11. A recombinant DNA sequence of claim 1 comprising a cDNA sequence encoding pre-pro-beta-1,3-glucanase.

12. A recombinant DNA sequence of claim 1 comprising a 5' end coding sequence fragment of a cDNA sequence encoding pre-pro-beta-1,3-glucanase.

13. A recombinant DNA sequence of claim 12 wherein the coding sequence fragment comprises at least 800 nucleotides.

14. A recombinant DNA sequence of claim 5 comprising a cDNA sequence encoding mature tobacco beta-1,3-glucanase.

15. A recombinant DNA sequence of claim 5 comprising a cDNA sequence encoding tobacco pre-pro-beta-1 ,3-glucanase.

16. A recombinant DNA sequence of claim 1 comprising a DNA sequence selected from the group consisting of Sequence DNA-I, Sequence DNA-III, and Sequence DNA-IV.

17. The genomic DNA sequence of claim 7 which is the DNA of Sequence V.

18. The genomic DNA sequence of claim 7 which is the DNA of Sequence VI.

19. A recombinant DNA sequence of claim 5 encoding a polypeptide of the formula 
Q S I G V C Y G M L G N N L P N H W
E V I Q L Y K S R N I G R L R L Y D P N
H G A L Q A L K G S N I E V M L G L P N
S D V K H I A S G M E H A R W W V Q K N
V K D F W P D V K I K Y I A V G N E I S
P V T G T S Y L T S F L T P A M V N I Y
K A I G E A G L G N N I K V S T S V D M
T L I G N S Y P P S Q G S F R N D A R W
F V/T D P I V G F L R D T R A P L L V N I
Y P Y F S Y S G N P G Q I S L P Y S L F
T A P N V V V Q D G S R Q Y R N L F D A
M L D S V Y A A L E R S G G A S V G I V
 
V S E S G W P S A G A F G A T Y D N A A
T Y L R N/Y L I Q/P H A K E G S P R K P G P
I E T Y I F A M F D E N N K N P E L E K
H F G L F S P N K Q P K Y N I/L N F G
 wherein letters separated by a slash represent either amino acid.

20. A recombinant DNA sequence of claim 5 encoding a polypeptide of the formula 
M S T S H K H N T P
Q M A A I T L L G L L L V A S S/T I D/E I A
G A Q S I G V C Y G M L G N N L P N H W
E V I Q L Y K S R N I G R L R L Y D P N
H G A L Q A L K G S N I E V M L G L P N
S D V K H I A S G M E H A R W W V Q K N
V K D F W P D V K I K Y I A V G N E I S
P V T G T S Y L T S F L T P A M V N I Y
K A I G E A G L G N N I K V S T S V D M
T L I G N S Y P P S Q G S F R N D A R W
F V/T D P I V G F L R D T R A P L L V N I
Y P Y F S Y S G N P G Q I S L P Y S L F
T A P N V V V Q D G S R Q Y R N L F D A
M L D S V Y A A L E R S G G A S V G I V
V S E S G W P S A G A F G A T Y D N A A
T Y L R N/Y L I Q/P H A K E G S P R K P G P
I E T Y I F A M F D E N N K N P E L E K
H F G L F S P N K Q P K Y N I/L N F G V S
G G V W D S S V E T N A T A S L V/I S E M
 wherein letters separated by a slash represent either amino acid.

21. A signal peptide of tobacco beta-1,3-glucanase having the formula 
M S T S H K H N T P Q M A A I T L L G L
L L V A S S/T I D/E I A G A
 wherein letters separated by a slash represent either amino acid.

22. A recombinant DNA sequence encoding a peptide of the formula 
M S T S H K H N T P Q M A A I T L L G L
L L V A S S/T I D/E I A G A
 wherein letters separated by a slash represent either amino acid.

## Patentansprüche

1. Rekombinante DNA-Sequenz, umfassend eine DNA-Sequenz, die ein Polypeptid mit β-1,3-Glucanaseaktivität codiert, die eine wesentliche Sequenzhomologie mit einer DNA-Sequenz, ausgewählt aus der Gruppe, bestehend aus Sequenz DNA-I, Sequenz DNA-III, Sequenz DNA-IV, Sequenz-V, Sequenz VI, Sequenz DNA-VII und Sequenz DNA-VIII, besitzt.

2. Rekombinante DNA-Sequenz nach Anspruch 1, wobei die DNA-Sequenz, die ein Polypeptid mit β-1,3-Glucanaseaktivität codiert, ein molekulares Fragment aus genomischer DNA ist.

3. Rekombinante DNA-Sequenz nach Anspruch 1, wobei die DNA-Sequenz, die ein Polypeptid mit β-1,3-Glucanaseaktivität codiert, eine aus mRNA oder einem mRNA-Fragment hergestellte cDNA oder eine DNA-Sequenz mit einer wesentlichen Sequenzhomologie mit einer solchen cDNA ist.

4. Rekombinante DNA-Sequenz nach Anspruch 1, die eine cDNA, hergestellt aus mRNA oder aus einem mRNA-Fragment, oder eine DNA-Sequenz mit einer wesentlichen Sequenzhomologie mit einer solchen cDNA ist.

5. Rekombinante DNA-Sequenz nach Anspruch 1, die Tabak-β-1,3-Glucanase codiert.

6. Gereinigte genomische DNA-Sequenz, die ein Polypeptid mit β-1,3-Glucanaseaktivität codiert, die eine wesentliche Sequenzhomologie zu einer DNA-Sequenz, ausgewählt aus der Gruppe, bestehend aus Sequenz V und Sequenz VI, besitzt.

7. Gereinigte genomische DNA-Sequenz nach Anspruch 6, wobei die β-1,3-Glucanase Tabak-β-1,3-glucanase ist.

8. Rekombinante DNA-Sequenz nach Anspruch 1, umfassend eine cDNA-Sequenz, die reife β-1,3-Glucanase codiert.

9. Rekombinante DNA-Sequenz nach Anspruch 1, umfassend ein 5'-Ende-Fragment der codierenden Sequenz einer cDNA-Sequenz, die reife β-1,3-Glucanase codiert.

10. Rekombinante DNA-Sequenz nach Anspruch 9, wobei das codierende Sequenz-Fragment mindestens 700 Nucleotide umfaßt.

11. Rekombinante DNA-Sequenz nach Anspruch 1, umfassend eine cDNA-Sequenz, die Präpro-β-1,3-glucanase codiert.

12. Rekombinante DNA-Sequenz nach Anspruch 1, umfassend ein 5'-Ende-Fragment der codierenden Sequenz einer cDNA-Sequenz, die Präpro-β-1,3-glucanase codiert.

13. Rekombinante DNA-Sequenz nach Anspruch 12, wobei das codierende Sequenz-Fragment mindestens 800 Nucleotide umfaßt.

14. Rekombinante DNA-Sequenz nach Anspruch 5, umfassend eine cDNA-Sequenz, die reife Tabak-β-1,3-glucanase codiert.

15. Rekombinante DNA-Sequenz nach Anspruch 5, umfassend eine cDNA-Sequenz, die Tabak-präpro-β-1,3-glucanase codiert.

16. Rekombinante DNA-Sequenz nach Anspruch 1, umfassend eine DNA-Sequenz, ausgewählt aus der Gruppe, bestehend aus Sequenz DNA-I, Sequenz DNA-III und Sequenz DNA-IV.

17. Genomische DNA-Sequenz nach Anspruch 7, welche die DNA von Sequenz V ist.

18. Genomische DNA-Sequenz nach Anspruch 7, welche die DNA von Sequenz VI ist.

19. Rekombinante DNA-Sequenz nach Anspruch 5, codierend ein Polypeptid der Formel 
Q S I G V C Y G M L G N N L P N H W
E V I Q L Y K S R N I G R L R L Y D P N
H G A L Q A L K G S N I E V M L G L P N
S D V K H I A S G M E H A R W W V Q K N
V K D F W P D V K I K Y I A V G N E I S
P V T G T S Y L T S F L T P A M V N I Y
K A I G E A G L G N N I K V S T S V D M
T L I G N S Y P P S Q G S F R N D A R W
F V/T D P I V G F L R D T R A P L L V N I
Y P Y F S Y S G N P G Q I S L P Y S L F
T A P N V V V Q D G S R Q Y R N L F D A
M L D S V Y A A L E R S G G A S V G I V
V S E S G W P S A G A F G A T Y D N A A
T Y L R N/Y L I Q/P H A K E G S P R K P G P
I E T Y I F A M F D E N N K N P E L E K
H F G L F S P N K Q P K Y N I/L N F G
 wobei die durch einen Schrägstrich getrennten Buchstaben beide Aminosäuren bedeuten.

20. Rekombinante DNA-Sequenz nach Anspruch 5, codierend ein Polypeptid der Formel 
M S T S H K H N T P
Q M A A I T L L G L L L V A S S/T I D/E I A
G A Q S I G V C Y G M L G N N L P N H W
E V I Q L Y K S R N I G R L R L Y D P N
H G A L Q A L K G S N I E V M L G L P N
S D V K H I A S G M E H A R W W V Q K N
V K D F W P D V K I K Y I A V G N E I S
P V T G T S Y L T S F L T P A M V N I Y
K A I G E A G L G N N I K V S T S V D M
T L I G N S Y P P S Q G S F R N D A R W
F V/T D P I V G F L R D T R A P L L V N I
Y P Y F S Y S G N P G Q I S L P Y S L F
T A P N V V V Q D G S R Q Y R N L F D A
M L D S V Y A A L E R S G G A S V G I V
V S E S G W P S A G A F G A T Y D N A A
T Y L R N/Y L I Q/P H A K E G S P R K P G P
I E T Y I F A M F D E N N K N P E L E K
H F G L F S P N K Q P K Y N I/L N F G V S
G G V W D S S V E T N A T A S L V/I S E M
 wobei die durch einen Schrägstrich getrennten Buchstaben beide Aminosäuren bedeuten.

21. Signalpeptid von Tabak-β-1,3-glucanase mit der Formel 
M S T S H K H N T P Q M A A I T L L G L
L L V A S S/T I D/E I A G A
 wobei die durch einen Schrägstrich getrennten Buchstaben beide Aminosäuren bedeuten.

22. Rekombinante DNA-Sequenz, die ein Peptid der Formel 
M S T S H K H N T P Q M A A I T L L G L
L L V A S S/T I D/E I A G A
 codiert, wobei die durch einen Schrägstrich getrennten Buchstaben beide Aminosäuren bedeuten.

## Revendications

1. Séquence d'ADN recombinant comprenant une séquence d'ADN codant pour un polypeptide ayant une activité de bêta-1,3-glucanase qui a une homologie de séquence substantielle à une séquence d'ADN choisie dans le groupe constitué par la Séquence d'ADN-I, la Séquence d'ADN III, la Séquence d'ADN-IV, la Séquence d'ADN-V, la Séquence d'ADN-VI, la Séquence d'ADN-VII et la Séquence d'ADN-VIII.

2. Séquence d'ADN recombinant de la revendication 1, où ladite séquence d'ADN codant pour un polypeptide ayant une activité de bêta-1,3-glucanase est un fragment moléculaire de l'ADN génomique.

3. Séquence d'ADN recombinant de la revendication 1, où ladite séquence d'ADN codant pour un polypeptide ayant une activité de bêta-1,3-glucanase est un ADN préparé à partir de l'ARNm ou à partir d'un fragment d'ARNm, ou une séquence d'ADN ayant une homologie de séquence substantielle à un tel ADNc.

4. Séquence d'ADN recombinant de la revendication 1, qui est un ADNc préparé à partir de l'ARNm ou à partir d'un fragment d'ARNm, ou une séquence d'ADN ayant une homologie de séquence substantielle à un tel ADNc.

5. Séquence d'ADN recombinant de la revendication 1 codant pour la bêta-1,3-glucanase.

6. Séquence d'ADN génomique purifié codant pour un polypeptide ayant une activité de bêta-1,3-glucanase qui a une homologie de séquence substantielle à une séquence d'ADN choisie dans le groupe constitué par la Séquence V et la Séquence VI.

7. Séquence d'ADN génomique purifiée de la revendication 6, dans laquelle ladite β-1,3-glucanase est la bêta-1,3-glucanase du tabac.

8. Séquence d'ADN recombinant de la revendication 1, comprenant une séquence d'ADNc codant pour la bêta-1,3-glucanase mature.

9. Séquence d'ADN recombinant de la revendication 1, comprenant un fragment de séquence du codage d'extrémité 5' d'une séquence d'ADNc codant pour la bêta-1,3-glucanase mature.

10. Séquence d'ADN recombinant de la revendication 9, dans laquelle le fragment de séquence de codage comprend au moins 700 nucléotides.

11. Séquence d'ADN recombinant de la revendication 1, comprenant une séquence d'ADN codant pour la pré-pro-bêta-1,3-glucanase.

12. Séquence d'ADN recombinant de la revendication 1, comprenant un fragment de séquence de codage d'extrémité 5' d'une séquence d'ADNc codant pour la pré-pro-bêta-1,4-glucanase.

13. Séquence d'ADN recombinant de la revendication 12, dans laquelle le fragment de séquence de codage comprend au moins 800 nucléotides.

14. Séquence d'ADN recombinant de la revendication 15, comprenant une séquence d'ADNc codant pour la bêta-1,3-glucanase du tabac mature.

15. Séquence d'ADN recombinant de la revendication 5, comprenant une séquence d'ADNc codant pour la pré-pro-bêta-1,3-glucanase du tabac.

16. Séquence d'ADN recombinant de la revendication 1, comprenant une séquence d'ADN choisie dans le groupe constitué de la Séquence d'ADN-I, la Séquence d'ADN-III et la Séquence d'ADN-IV.

17. Séquence d'ADN génomique de la revendication 7, qui est l'ADN de la Séquence V.

18. Séquence d'ADN génomique de la revendication 7, qui est l'ADN de la Séquence VI.

19. Séquence d'ADN recombinant de la revendication 5, codant pour un polypeptide de formule 
Q S I G V C Y G M L G N N L P N H W
E V I Q L Y K S R N I G R L R L Y D P N
H G A L Q A L K G S N I E V M L G L P N
S D V K H I A S G M E H A R W W V Q K N
V K D F W P D V K I K Y I A V G N E I S
P V T G T S Y L T S F L T P A M V N I Y
 
K A I G E A G L G N N I K V S T S V D M
T L I G N S Y P P S Q G S F R N D A R W
F V/T D P I V G F L R D T R A P L L V N I
Y P Y F S Y S G N P G Q I S L P Y S L F
T A P N V V V Q D G S R Q Y R N L F D A
M L D S V Y A A L E R S G G A S V G I V
V S E S G W P S A G A F G A T Y D N A A
T Y L R N/Y L I Q/P H A K E G S P R K P G P
I E T Y I F A M F D E N N K N P E L E K
H F G L F S P N K Q P K Y N I/L N F G
 où les lettres séparées par une barre oblique représentent l'un ou l'autre acide aminé.

20. Séquence d'ADN recombinant de la revendication 5, codant pour un polypeptide de formule: 
M S T S H K H N T P
Q M A A I T L L G L L L V A S S/T I D/E I A
G A Q S I G V C Y G M L G N N L P N H W
E V I Q L Y K S R N I G R L R L Y D P N
H G A L Q A L K G S N I E V M L G L P N
S D V K H I A S G M E H A R W W V Q K N
V K D F W P D V K I K Y I A V G N E I S
P V T G T S Y L T S F L T P A M V N I Y
K A I G E A G L G N N I K V S T S V D M
T L I G N S Y P P S Q G S F R N D A R W
F V/T D P I V G F L R D T R A P L L V N I
Y P Y F S Y S G N P G Q I S L P Y S L F
T A P N V V V Q D G S R Q Y R N L F D A
M L D S V Y A A L E R S G G A S V G I V
V S E S G W P S A G A F G A T Y D N A A
T Y L R N/Y L I Q/P H A K E G S P R K P G P
I E T Y I F A M F D E N N K N P E L E K
H F G L F S P N K Q P K Y N I/L N F G V S
G G V W D S S V E T N A T A S L V/I S E M
 où les lettes séparées par une barre oblique représentent l'un ou l'autre acide aminé.

21. Peptide signal de la bêta-1,3-glucanase ayant la formule 
M S T S H K H N T P Q M A A I T L L G L
L L V A S S/T I D/E I A G A
 où les lettres séparées par une barre oblique représentent l'un ou l'autre acide aminé.

22. Séquence d'ADN recombinant codant pour un peptide de formule 
M S T S H K H N T P Q M A A I T L L G L
L L V A S S/T I D/E I A G A
 où les lettres séparées par une barre oblique représentent l'un ou l'autre acide aminé.
